# EUROPEAN PATENT SPECIFICATION

(11) **EP 3 878 954 B1**
(45) Date of publication and mention of the grant of the patent: **02.09.2026**
(21) Application number: 18939347.3
(22) Date of filing: 07.11.2018
(51) Int. Cl.: C12N 9/10, C12N 15/54, C12N 15/63, C12N 15/52, C12N 15/70

(54) **ENZYME GENE FOR BIOSYNTHESIS OF CROCIN AND USE THEREOF**
ENZYMGEN ZUR BIOSYNTHESE VON CROCIN UND VERWENDUNG DAVON
GÈNE D'ENZYME POUR LA BIOSYNTHÈSE DE CROCINE ET UTILISATION CORRESPONDANTE

(43) Date of publication of application: 15.09.2021
(73) Proprietor: Institute of Medicinal Plant Development, Chinese Academy of Medical Sciences, Beijing 100193 (CN)
(72) Inventor: SONG, Jingyuan, Beijing 100193 (CN); XU, Zhichao, Beijing 100193 (CN); PU, Xiangdong, Beijing 100193 (CN); GAO, Ranran, Beijing 100193 (CN)
(74) Representative: Kutzenberger Wolff & Partner
(86) International application number: PCT/CN2018/114419
(87) International publication number: WO 2020/093285

(56) References cited:
- WO-A2-2013/021261
- US-A1- 2017 044 552
- JI AIJIA ET AL: "Transcriptome-Guided Mining of Genes Involved in Crocin Biosynthesis", FRONTIERS IN PLANT SCIENCE, vol. 8, 11 April 2017 (2017-04-11), XP055928546, DOI: 10.3389/fpls.2017.00518
- DATABASE UniProt [online] 1 October 2014 (2014-10-01), "RecName: Full=Glycosyltransferase {ECO:0000256|RuleBase:RU362057}; EC=2.4.1.- {ECO:0000256|RuleBase:RU362057};", retrieved from EBI accession no. UNIPROT:A0A068U609 Database accession no. A0A068U609
- DATABASE EMBL [online] 27 June 2014 (2014-06-27), "Coffea canephora hypothetical protein ID - CDP03995; SV 1; linear; genomic DNA; CON; PLN; 1380 BP.", retrieved from EBI accession no. EMBL:CDP03995
- "Unnamed Protein Product [Coffea Canephora", GENBANK: CDP06918.1, 27 June 2014 (2014-06-27), XP055706440
- "Unnamed Protein Product [Coffea Canephora", GENBANK: CDP03995.1, 27 June 2014 (2014-06-27), XP055706442
- DENOEUD, F. ET AL.: "The Coffee Genome Provides Insight into the Convergent Evolution of Caffeine Biosynthesis", SCIENCE, vol. 345, no. 6201, 27 June 2014 (2014-06-27), XP055706444, ISSN: 1095-9203

## Description

### Technical Field

The present invention relates to the field of biotechnology, in particular to an enzyme gene for biosynthesis of crocin and use thereof. The invention is set out in the appended claims.

### Background Art

The current edition of The Pharmacopoeia of the People's Republic of China recites that Croci Stigma (Xihonghua) is the dried stigma of *Crocus sativus* L., a plant of Iridaceae. Croci Stigma is known as "red gold" due to its complicated harvesting steps, extremely high labor cost, low yield and high value. The main active ingredients of Croci Stigma are crocins, which have pharmacological effects of antidepression, anti-inflammation, anti-tumor and the like.

The Gardeniae Fructus (Zhizi) included in the Chinese Pharmacopoeia belongs to the dried and mature fruit of *Gardenia jasminoides,* a plant of Rubiaceae. Gardenia fruit is rich in crocins, and the main active ingredients of the gardenia fruit are geniposides and crocins. Gardeniae Fructus is considered to be an ideal alternative resource for crocins because of its rich resources and high yield. The biosynthetic route of crocins is mainly composed of three parts: 1. carotenoid cleavage dioxygenase cleaves carotenoid substances to produce crocetin dialdehyde; 2. crocetin dialdehyde is catalyzed by acetaldehyde dehydrogenase to produce crocetin; and 3. crocetin is subjected to the glycosylation of glycosyltransferase to produce different crocins I-V, the main difference between crocins I-V lies in the number of glycosides. The present invention lays a foundation for the synthetic biology research of crocin by analyzing the genome of *Gardenia jasminoides,* mining the key enzyme gene for crocin synthesis, and analyzing the biosynthetic route of crocin.

### Summary of the Invention

The present invention aims to provide a key enzyme gene for biosynthesis of crocins. The amino acid sequence of a protein encoded by the enzyme gene is as shown in SEQ ID NO. 8, or is a sequence having at least 92% identity to the amino acid sequence as shown in SEQ ID NO. 8.

Preferably, the amino acid sequence of the protein encoded by the enzyme gene is as shown in SEQ ID NO. 8, or is a sequence having at least 92% identity, at least 95% identity, or at least 98% identity to the amino acid sequence as shown in SEQ ID NO. 8.

Preferably, the nucleotide sequence of the enzyme gene is as shown in SEQ ID NO. 4, or is a sequence having at least 92% identity to the nucleotide sequence as shown in SEQ ID NO. 4. The SEQ ID NO. 4 is GjUGT74F8.

Preferably, the nucleotide sequence of the enzyme gene is as shown in SEQ ID NO. 4, or is a sequence having at least 92% identity, at least 95% identity, or at least 98% identity to the nucleotide sequence as shown in SEQ ID NO. 4.

The term "identity" describes the degree of similarity between two or more nucleotide sequences or amino acid sequences when used in reference to nucleic acids or amino acids. The percentage of identity between two sequences can be determined by comparing two best aligned sequences on a comparison window such that the sequence portion in the comparison window may contain substitutions, deletions, or additions (gaps) relative to the reference sequence (which does not contain additions or deletions) in order to achieve the best alignment of the two sequences.

The present invention also provides a biomaterial, containing an enzyme gene which has a nucleotide sequence as shown in SEQ ID NO. 4 or an amino acid sequence encoded as shown in SEQ ID NO. 8, or an enzyme gene which has a nucleotide sequence having at least 92% identity to the nucleotide sequence as shown in SEQ ID NO. 4, or an enzyme gene which has an amino acid sequence having at least 92% identity to the amino acid sequence as shown in SEQ ID NO. 8.

The SEQ ID NOs. 1-3 are GjCCD4a, GjALDH2C3, and GjUGT94E13, respectively.

The biomaterial is an expression vector, a cell, a cell line, an expression cassette or engineering bacteria.

The expression vector in the present invention refers to any vector known in the prior art that enables expression in microorganisms. Preferably, the expression vectors are prokaryotic expression vectors pET32a, pET28a and pCold I serial vectors, and eukaryotic expression vector pESC serial vectors.

Preferably, the cell is *Escherichia coli* cell suitable for prokaryotic expression vectors or *Saccharomyces cerevisiae* cell suitable for eukaryotic expression vectors.

Preferably, the engineering microbes are one or more of *Escherichia coli* and *Saccharomyces cerevisiae.*

The present invention also provides a CCD protein, and the CCD protein is encoded by the nucleotide sequence as shown in SEQ ID NO. 1 or is obtained from the amino acid sequence as shown in SEQ ID NO. 5. The CCD protein is a carotenoid cleavage dioxygenase which can perform specific cleavage at the 7, 8 (7', 8') double bond position of zeaxanthin, lycopene, and β-carotene to produce crocetin dialdehyde. This CCD protein is not literally claimed.

The present invention also provides an ALDH protein, and the ALDH protein is encoded by the nucleotide sequence as shown in SEQ ID NO. 2 or is obtained from the amino acid sequence as shown in SEQ ID NO. 6. The ALDH protein is an acetaldehyde dehydrogenase which can catalyze the synthesis of crocetin from crocetin dialdehyde. This ALDH protein is not literally claimed.

The present invention also provides two UGT proteins, and the UGT proteins are encoded by the nucleotide sequences as shown in SEQ ID NO. 3 or 4 or is obtained from the amino acid sequence as shown in SEQ ID NO. 7 or 8. These two UGT proteins are glycosyltransferases which can glycosylate crocetin to synthesize crocins I-V. The claimed UGT protein is obtained from the amino acid sequence as shown in SEQ ID NO. 8.

The present invention also provides the use of an enzyme gene of which the nucleotide sequence is as shown in SEQ ID NO. 4 or by which the amino acid sequence of a protein encoded is as shown in SEQ ID NO. 8, or the enzyme gene of which the nucleotide sequence has at least 92% identity to the nucleotide sequence as shown in SEQ ID NO. 4, or the enzyme gene by which the amino acid sequence of a protein encoded has at least 92% identity to the amino acid sequence as shown in SEQ ID NO. 8, or the above-mentioned biomaterial, or the protein encoded by the nucleotide sequence as shown in SEQ ID NO. 4 or the amino acid sequence as shown in SEQ ID NO. 8, in the microbial industrial synthesis of crocins.

Preferably, the use is the application of a CCD protein encoded by the nucleotide sequence as shown in SEQ ID NO. 1 or obtained from the amino acid sequence as shown in SEQ ID NO. 5 in specific cleavage at the 7, 8 (7', 8') double bond position of zeaxanthin, lycopene, and β-carotene to produce crocetin dialdehyde.

Preferably, the use is the application of an ALDH protein encoded by the nucleotide sequence as shown in SEQ ID NO. 2 or obtained from the amino acid sequence as shown in SEQ ID NO. 6 in catalyzing the synthesis of crocetin from crocetin dialdehyde.

Preferably, the use is the application of a UGT protein encoded by the nucleotide sequence as shown in SEQ ID NO. 3 or 4 or obtained from the amino acid sequence as shown in SEQ ID NO. 7 or 8 in the synthesis of crocins I-V from crocetin by glycosylation.

Based on the *Gardenia jasminoides* genome and transcriptome, the present invention successfully analyzes the biosynthetic route of crocins. Therefore, the present invention also provides a synthesis method of crocins, and the method comprises the following steps:
1) using a CCD protein encoded by the nucleotide sequence as shown in SEQ ID NO. 1 or obtained from the amino acid sequence as shown in SEQ ID NO. 5 to cleave zeaxanthin, lycopene, or β-carotene to produce crocetin dialdehyde;
2) using an ALDH protein encoded by the nucleotide sequence as shown in SEQ ID NO. 2 or obtained from the amino acid sequence as shown in SEQ ID NO. 6 to dehydrogenate the crocetin dialdehyde to produce crocetin; and
3) using a UGT protein encoded by the nucleotide sequence as shown in SEQ ID NO. 3 or 4 or obtained from the amino acid sequence as shown in SEQ ID NO. 7 or 8 to glycosylate crocetin to produce crocins. This method is not literally claimed.

### Synopsis

The present disclosure obtains the enzyme gene of the present disclosure by screening the key enzyme gene in the biosynthetic route of crocins in *Gardenia jasminoides,* and the amino acid sequence of the enzyme gene is as shown in any one of SEQ ID NOs. 5-8, or is a sequence having at least 92% identity to the amino acid sequence as shown in any one of SEQ ID NOs. 5-8, or the nucleotide sequence of the enzyme gene is as shown in any one of SEQ ID NO. 1-4, or is a sequence having at least 92% identity to the nucleotide sequence as shown in SEQ ID NOs. 1-4. The sequence of the enzyme gene of the present disclosure can be used to synthesize crocins in microorganisms, effectively increase the yield and purity of crocins, reduce the cost of obtaining crocins, and has high application value.

### Brief Description of the Drawings

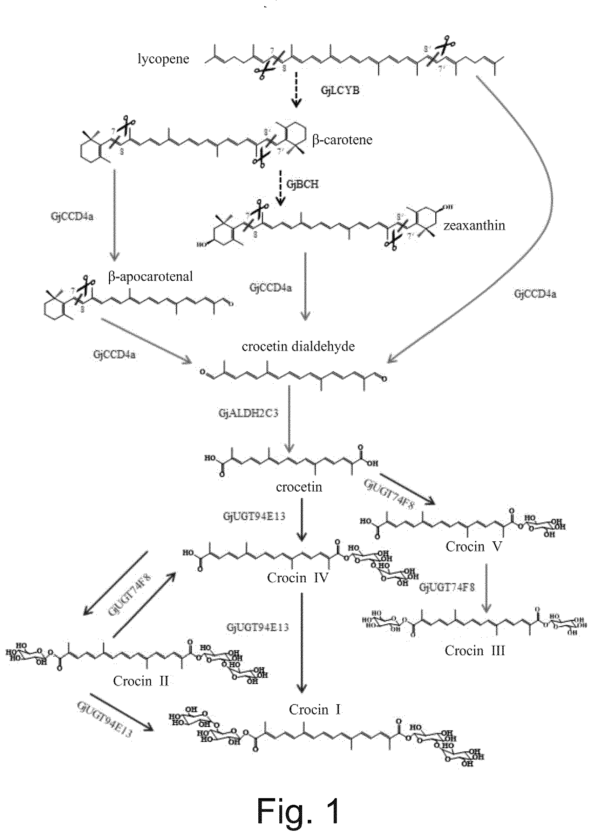

Figure 1 shows the biosynthetic route of crocins in *Gardenia jasminoides.*

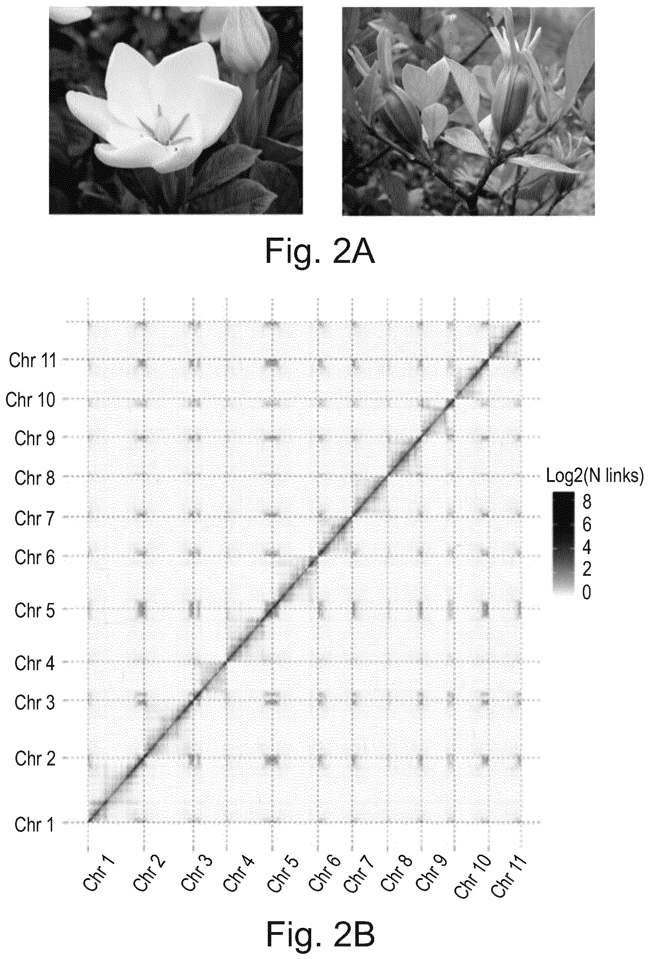

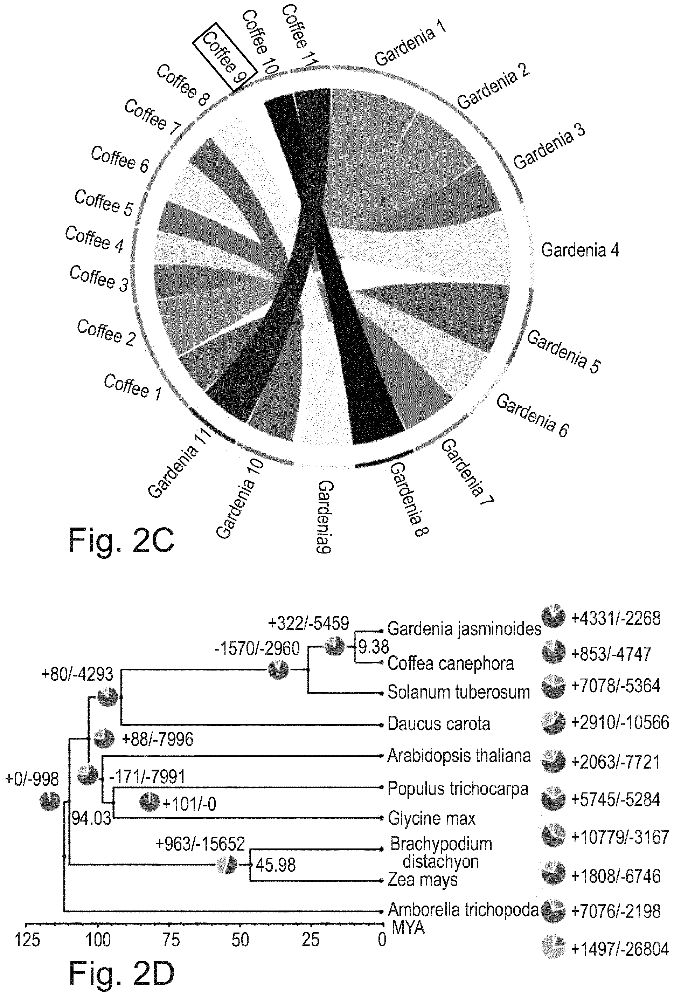

Figure 2 shows the assembly and annotation results of the *Gardenia jasminoides* genome.

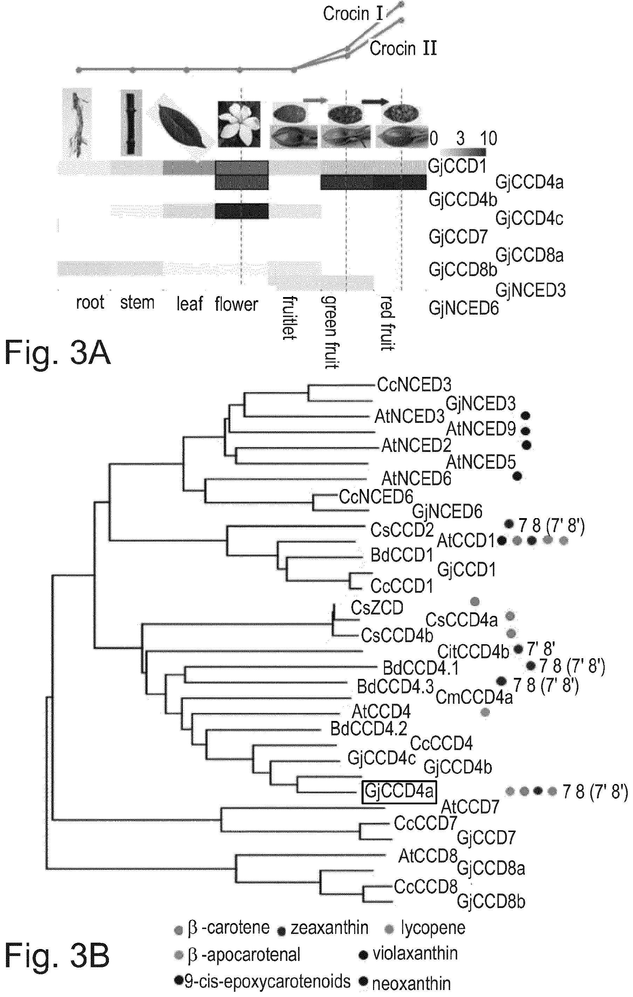

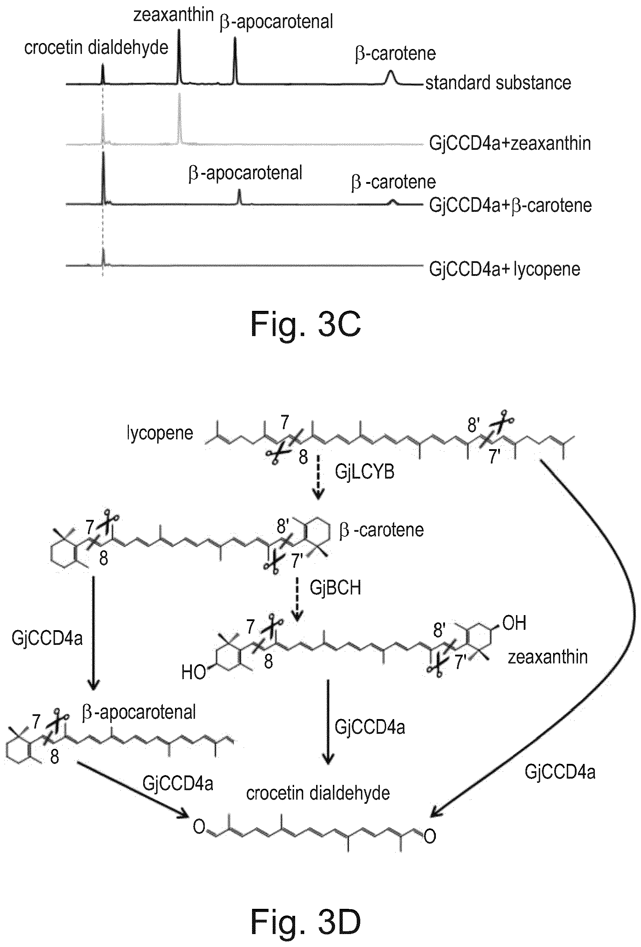

Figure 3 shows the screening and functional identification of GjCCD involved in the synthesis of crocetin dialdehyde in *Gardenia jasminoides.*

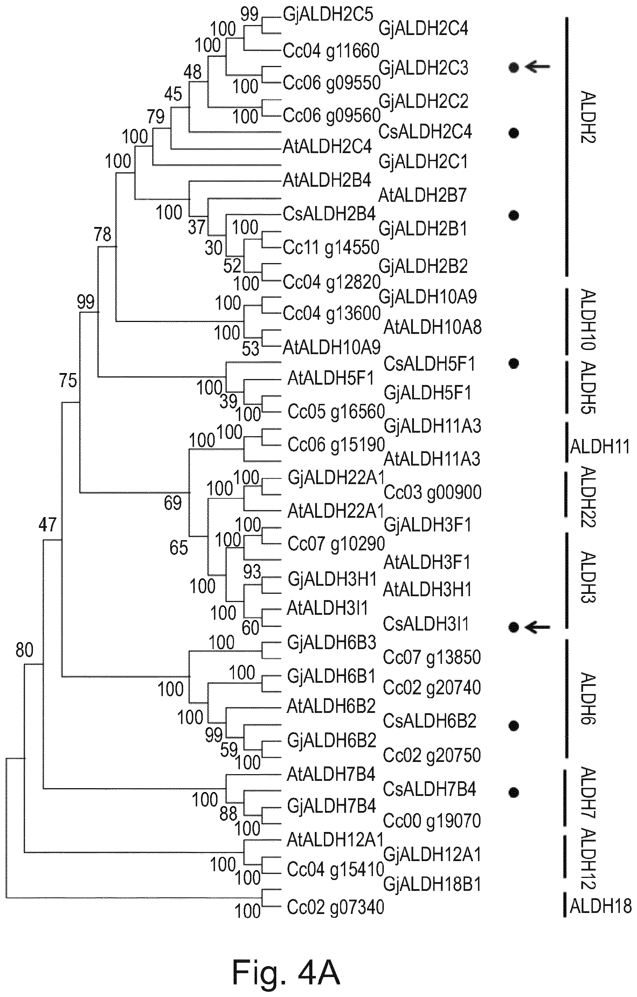

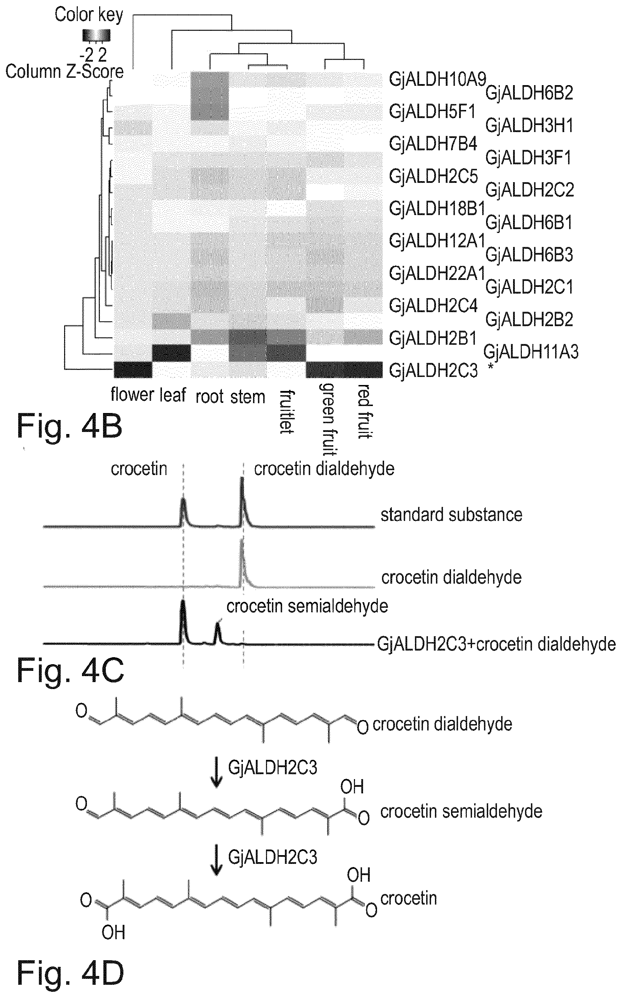

Figure 4 shows the screening and functional identification of GjALDH involved in the synthesis of crocetin in *Gardenia jasminoides.*

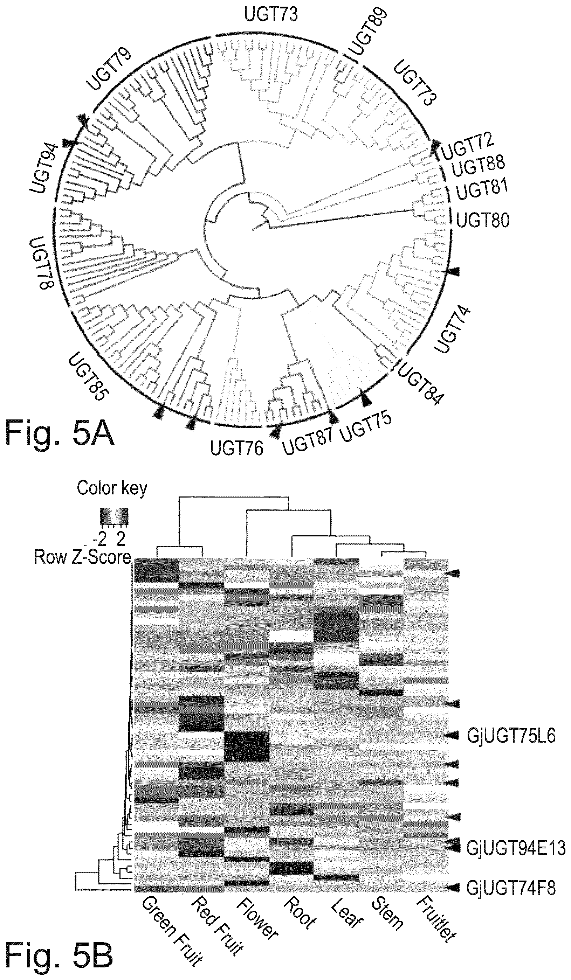

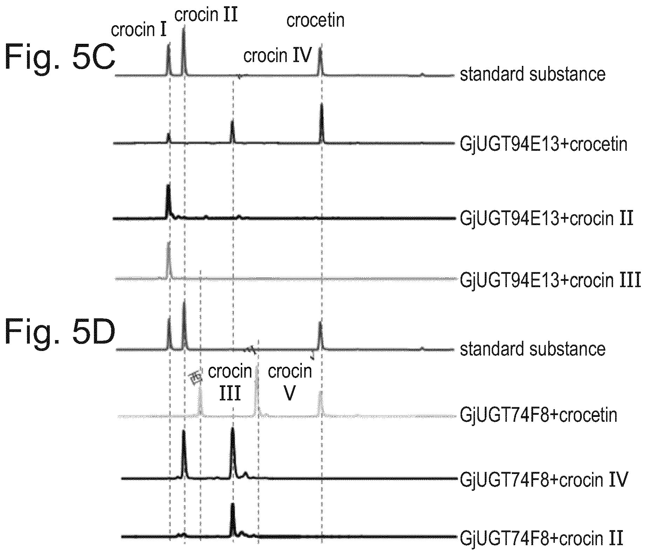

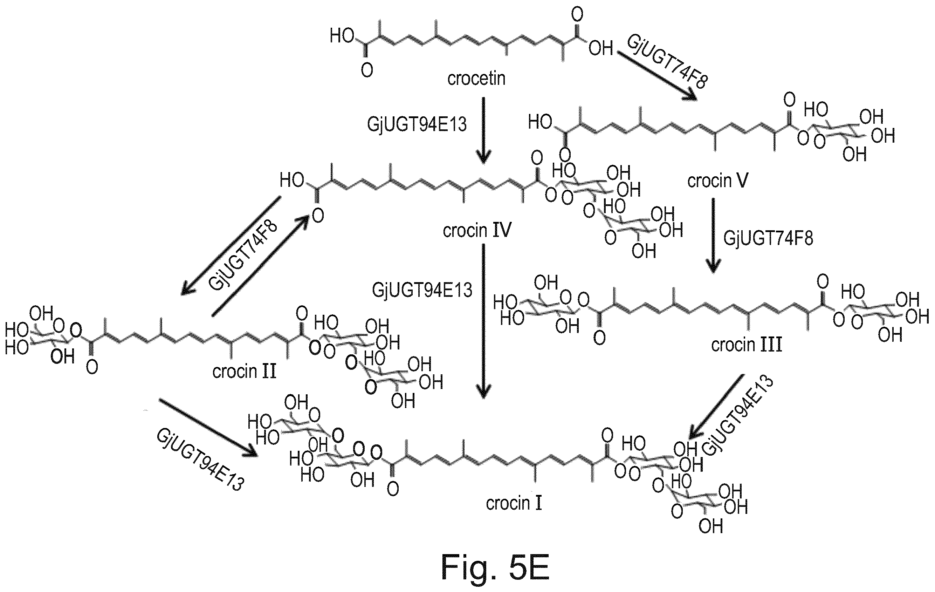

Figure 5 shows the screening and functional identification of GjUGTs involved in the synthesis of crocins in *Gardenia jasminoides.*

### Specific Modes for Carrying Out the Embodiments

The technical solutions in the examples of the present invention will be clearly and completely described below. Obviously, the described examples are only a part of the embodiments, rather than all embodiments of the present invention. Based on the examples of the present invention, all other embodiments obtained by a person skilled in the art without creative efforts shall fall within the protection scope of the claims, as defined by the claims.

### Example 1: Gene mining in biosynthetic route of crocins based on Gardenia jasminoides genome and transcriptome data

### 1) Gardenia jasminoides genome assembly using the combination of ONT, NGS sequencing and Hi-C technology

The results of the *Gardenia jasminoides* Genome Survey show that the *Gardenia jasminoides* genome is highly heterozygous. In the present Example, long-read sequencing of nanopore sequencing ONT (Oxford Nanopore Technology) was performed the assembly of *Gardenia jasminoides* genome with high heterozygosity and high repetitive sequence. The 60 × ONT sequencing data was corrected by CANU software and assembled by SMARTdenovo software. The total length of the finally assembled *Gardenia jasminoides* genome was 681.4 Mb, and the genome sequence fragment N50 reached 1.1 Mb. The integrity of the *Gardenia jasminoides* genome evaluated by BUSCO was 92.8%. *Gardenia jasminoides* is diploid and the number of chromosomes is 2n=22. Using chromosome conformation capture technology, the *Gardenia jasminoides* genome was assembled at chromosome level. The genome size was 630 Mb, and the genome sequence fragment N50 reached 51.6 Mb.

### 2) Detection of crocin content in roots, stems, leaves, flowers, fruitlets, green fruits and red fruits of Gardenia jasminoides.

The present Example intends to determine the difference in crocin content in different tissue parts of *Gardenia jasminoides.* After drying, the roots, stems, leaves, flowers, fruitlets, green fruits, and red fruits of *Gardenia jasminoides* were ground into fine powder and sieved. Each sample of 0.25 g was accurately weighed into a triangular flask, 25 mL of 50% chromatography grade methanol was added, the volume was calibrated, ultrasonic extraction was performed for 30 minutes, and the resultant stand to perform cooling, then the volume was calibrated, 50% methanol was used to make up for the weight loss. The extract solution was filtered and passed through a 0.22 µm filter membrane to obtain a test sample. UPLC was used to detect the chemical composition of the test sample, with the instrument model of Thermo Ultimate 3000. The injection volume was 10 µL, the chromatographic column was: Waters Acquity UPLC^{®} BEH C18 column (1.7 µm, 100 × 2.1 mm), and the column temperature was: 30°C. The chromatographic conditions were as follows: UV 440 nm, mobile phase: (A): acetonitrile (containing 0.1% formic acid), (B): water (containing 0.1% formic acid), flow rate: 0.25 mL/min, elution procedure: 0 to 5 min, 10% A linearly increased to 40% A; 5 to 10 min, 40% A; 10 to 11 min, 40% A to 100%A, 11 to 13 min, 100% A.

The results of the present example showed that crocin was not detected in roots, stems, leaves, flowers and fruitlets of *Gardenia jasminoides,* and crocins were detected in green fruits and red fruits of *Gardenia jasminoides,* and the content of crocins in red fruits is higher than that in green fruits. The flesh of the fruitlet is all immature and is light brown. The flesh of the green fruit is partially mature and is light red. The flesh of the red fruit is all mature and is red. Studies have shown that crocins are specifically accumulated in the fruit, and as the fruit matures, the accumulation of crocin increases. The differential distribution of crocins in different parts provides an important basis for the screening of the key enzyme gene.

### 3) Screening candidate genes encoding key enzymes for synthesis of crocin based on tissue-specific expression

In the present example, the CCD, ALDH, and UGT gene family members of *Gardenia jasminoides* were analyzed by BLASTP algorithm, and were constructed corresponding phylogenetic trees. 9 GjCCDs (5 subfamilies), 19 GjALDHs (10 subfamilies) and 173 GjUGTs (15 subfamilies) were identified in *Gardenia jasminoides* genome. Transcriptome sequencing was performed on tissues of roots, stems, leaves, flowers, fruitlets, green fruits, and red fruits of *Gardenia jasminoides* by using RNA-Seq technology, sequencing fragments were compared to the *Gardenia jasminoides* genome using HiSAT2 software, and the expression amount (FPKM value) of annotated genes in *Gardenia jasminoides* genome in different tissues was calculated by Cufflinks software. GjCCD4a, GjALDH2C3 and 9 GjUGTs were screened as candidate genes related to synthesis of crocins based on tissue-specific expression.

### Example 2: specific cleavage of Gardenia jasminoides carotenoids with GjCCD4a to produce crocetin dialdehyde

### 1) specific cleavage of zeaxanthin with GjCCD4a to produce crocetin dialdehyde

The encoding gene sequence of GjCCD4a was cloned into prokaryotic expression vector pET32a, and co-transformed into *Escherichia coli* BL21 (DE3) host cells with plasmid pACCAR25ΔcrtX capable of producing zeaxanthin, screening was performed using ampicillin and chloramphenicol, and 0.3 mM IPTG was used for inducing at 15°C in dark for 24 h. Centrifugation was performed at 4°C, 7000 rpm for 15 min, the bacterial cells were extracted with acetone, the extract solution was subjected to vacuum drying, and finally dissolved with methanol. The catalysate was detected by the UPLC detection method same as that in Example 1. Studies confirmed that zeaxanthin catalyzed by GjCCD4a showed a new chromatographic peak at retention time of 9.25 min, and the peak appearance time and spectral information were consistent with those of crocetin dialdehyde.

The products extracted from the reaction were detected by Agilent Technologies 1290 Infinity II and 6545 Q-TOF LC/MS instrument, and subjected to qualitative analysis. The injection volume was 10 µL, the chromatographic column was: Waters Acquity UPLC^{®} BEH C18 column (1.7 µm, 100 × 2.1 mm), and the column temperature was: 30°C. The chromatographic conditions were as follows: UV 440 nm, mobile phase: (A): acetonitrile (containing 0.1% formic acid), (B): water (containing 0.1% formic acid), flow rate: 0.3 mL/min, elution procedure: 0 to 5 min, 10% A linearly increased to 50% A; 5 to 8 min, 50% A linearly increased to 90% A; 8-10 min, 90% A linearly increased to 100% A, 100% A was kept for one minute and then returned to the initial state. The 6545 Q-TOF mass spectrometer parameters were as follows: dry gas temperature of 325°C, flow rate of 6 L/min; sheath gas temperature of 350°C, flow rate of 12.0 L/min; sprayer pressure of 45 psig; and VCap of 4000 V. The mass spectrum information of the chromatographic peak generated by zeaxanthin catalyzed by GjCCD4a was [M+H]+: m/z 297.1939, which is consistent with crocetin dialdehyde. Therefore, the present Example verifies that GjCCD4a can perform specific cleavage at the 7, 8 (7', 8') double bond position of zeaxanthin and oxidize zeaxanthin into crocetin dialdehyde.

### 2) specific cleavage of lycopene with GjCCD4a to produce crocetin dialdehyde

The method was the same as 1) of Example 2. When the catalytic substrate was lycopene, GjCCD4a can specifically cleave lycopene at the 7, 8 (7', 8') double bond position of lycopene and oxidize lycopene into crocetin dialdehyde.

### 3) GjCCD4a specifically cleaves β-carotene to produce crocetin dialdehyde and its intermediate product

The method was the same as 1) of Example 2. When the catalytic substrate was β-carotene, GjCCD4a catalyzed β-carotene to produce two new chromatographic peaks, wherein, the spectrum and mass spectrum information of the chromatographic peak with a retention time of 9.25 min are consistent with those of crocetin dialdehyde, and the spectrum information of the chromatographic peak with a retention time of 13.50 min is consistent with the spectrum information of β-apocarotenal, and the nuclear magnetic information is the same. Therefore, GjCCD4a can specifically cleave β-carotene at the 7, 8 (7', 8') double bond position of β-carotene and oxidize β-carotene into crocetin dialdehyde, and the intermediate product β-apocarotenal was produced simultaneously.

### Example 3: efficient catalysis of crocetin dialdehyde with GjALDH2C3 to produce crocetin

The encoding gene of *GjALDH2C3* was cloned into pCold I vector to form pCold I-*GjALDH2C3* recombinant expression vector, and the vector was transformed into BL21 *Escherichia coli* expression strain. 2 mL of overnight induced bacterial solution was taken and added into 50 mL of LB liquid culture medium (resistant medium containing 50 µg/mL ampicillin), and activated at 37°C and 200 rpm for 2 to 3 h, low-temperature stimulation was performed at 15°C when the OD600 reached 0.4 to 0.5, and IPTG with a final concentration of 0.3 mM was added for inducing at 15°C and 130 rpm. After 24 hours of induction, centrifugation was performed to collect bacteria precipitate for protein purification, and the concentration was determined using the BCA protein kit.

The purified protein was functionally validated *in vitro* according to the following reaction system (50 µL): 100 mM Tris-HCl (pH 8.5), 50 µg purified protein, 1 mM NADP⁺, and 40 µM crocetin dialdehyde. After terminating the reaction with methanol, the resultant was passed through a 0.22 µm filter membrane, and its chemical composition was detected by UPLC and liquid chromatograph mass spectrometer (LC-MS/MS). The method was the same as 1) UPLC and mass spectrometry detection method of Example 2.

The *in vitro* catalysis results show that GjALDH2C3 can convert crocetin dialdehyde, the reaction produces 2 new chromatographic peaks, one of which has the same chromatographic behavior (such as retention time, spectrum and the like) as crocetin, the other has the chromatographic behavior between crocetin and crocetin dialdehyde, and the qualitative analysis of the catalysis products requires liquid chromatography-mass spectrometry detection.

### Example 4: glycosylation of crocetin with GjUGT94E5 and GjUGT74F8 to produce crocins

The method was the same as that of Example 3.

### 1) GjUGT94E13 function verification results and analysis

The liquid chromatography test results show that the protein has the following functions:
(1) catalyzing crocetin into crocin IV, which is further converted into crocin I;
(2) catalyzing crocin II into crocin I;
(3) catalyzing crocin III into crocin I.

### 2) GjUGT74F8 function verification results and analysis

(1) catalyzing crocetin into crocin V, which is further converted into crocin III;
(2) catalyzing crocin IV into crocin II;
(3) hydrolyzing crocin II into crocin IV.

Other candidate UGTs show no catalytic activity. The above descriptions are all judged on the basis of liquid chromatography detection, that is, materials have the same chromatographic behavior (such as retention time, spectrum and the like) compared with the standard products. The qualitative analysis of the compounds requires liquid chromatography-mass spectrometry detection.

### Industrial applicability [synopsis]

The present disclosure provides the key enzyme gene in the biosynthetic route of crocins. The nucleotide sequence of the enzyme gene is as shown in any one of SEQ ID NOs. 1-4 or is a sequence having at least 85% identity to the nucleotide sequence as shown in any one of SEQ ID NOs. 1-4. The present disclosure also provides a biomaterial containing the enzyme gene. The enzyme gene and the biomaterial described in the present disclosure are suitable for the industrial production of crocins in microorganisms. The sequence of the enzyme gene according to the present disclosure can synthesize crocins in microorganisms, effectively improve the yield and purity of crocins, and reduce the acquisition cost of crocins, with high application value and market prospect.

## Claims

1. An enzyme gene for biosynthesis of crocins, wherein the amino acid sequence of a protein encoded by the enzyme gene is as shown in SEQ ID NO. 8, or is a sequence having at least 92% identity to the amino acid sequence as shown in SEQ ID NO. 8.

2. The enzyme gene according to claim 1, wherein the nucleotide sequence of the enzyme gene is as shown in SEQ ID NO. 4, or is a sequence having at least 92% identity to the nucleotide sequence as shown in SEQ ID NO. 4.

3. A biomaterial being an expression vector, a cell, a cell line, an expression cassette, or an engineered microbe being one or more of *Escherichia coli* and *Saccharomyces cerevisiae,* containing an enzyme gene of which the nucleotide sequence is as shown in SEQ ID NO. 4 or by which the amino acid sequence of a protein encoded is as shown in SEQ ID NO. 8, or an enzyme gene of which the nucleotide sequence has at least 92% identity to the nucleotide sequence as shown in SEQ ID NO. 4, or an enzyme gene that encodes a protein that has at least 92% identity to the amino acid sequence as shown in SEQ ID NO. 8.

4. The biomaterial according to claim 3, wherein the expression vector is any one or more of prokaryotic expression vectors pET32a, pET28a and pCold I serial vectors, and eukaryotic expression vector pESC serial vectors.

5. The biomaterial according to claim 3, wherein the cell is *Escherichia coli* cell suitable for prokaryotic expression vectors or *Saccharomyces cerevisiae* cell suitable for eukaryotic expression vectors.

6. A UGT protein, wherein the UGT protein has the amino acid sequence as shown in SEQ ID NO. 8.

7. Use of the enzyme gene which has a nucleotide sequence as shown in SEQ ID NO. 4 or an amino acid sequence of a protein encoded as shown in SEQ ID NO. 8, or the enzyme gene which has a nucleotide sequence having at least 92% identity to the nucleotide sequence as shown in SEQ ID NO. 4, or the enzyme gene encoding a protein having at least 92% identity to the amino acid sequence as shown in SEQ ID NO. 8, or the biomaterial according to any one of claims 3-5, or the protein encoded by the nucleotide sequence as shown in SEQ ID NO. 4, in the microbial industrial synthesis of crocins.

8. The use according to claim 7, wherein the use is the application of a UGT protein having the amino acid sequence as shown in SEQ ID NO. 8 in the synthesis of crocins I-V from crocetin by glycosylation.

9. A synthesis method of crocins, wherein the method comprises the following step: using a UGT protein having the amino acid sequence as shown in SEQ ID NO. 8 to glycosylate the crocetin to produce crocin.

## Patentansprüche

1. Ein Enzymgen zur Biosynthese von Crocinen, wobei die Aminosäuresequenz eines durch das Enzymgen kodierten Proteins der in SEQ ID NO. 8 gezeigten entspricht oder eine Sequenz ist, die mindestens 92 % Identität mit der in SEQ ID NO. 8 gezeigten Aminosäuresequenz aufweist.

2. Das Enzymgen gemäß Anspruch 1, wobei die Nukleotidsequenz des Enzymgens der in SEQ ID NO. 4 gezeigten entspricht oder eine Sequenz ist, die mindestens 92 % Identität mit der in SEQ ID NO. 4 gezeigten Nukleotidsequenz aufweist.

3. Ein Biomaterial, das ein Expressionsvektor, eine Zelle, eine Zelllinie, eine Expressionskassette oder ein gentechnisch veränderter Mikroorganismus ist, der *Escherichia coli* und/oder *Saccharomyces cerevisiae* umfasst, und das ein Enzymgen enthält, dessen Nukleotidsequenz der in SEQ ID NO. 4 dargestellten entspricht, oder durch das die Aminosäuresequenz eines kodierten Proteins wie in SEQ ID NO. 8 dargestellt ist, oder ein Enzymgen enthält, dessen Nukleotidsequenz mindestens 92 % Identität mit der in SEQ ID NO. 4 gezeigten Nukleotidsequenz aufweist, oder ein Enzymgen enthält, das ein Protein kodiert, das mindestens 92 % Identität mit der in SEQ ID NO. 8 gezeigten Aminosäuresequenz aufweist.

4. Das Biomaterial nach Anspruch 3, wobei der Expressionsvektor einer oder mehrere der prokaryotischen Expressionsvektoren der pET32a-, pET28a- und pCold I-Serien sowie der eukaryotischen Expressionsvektoren der pESC-Serie ist.

5. Das Biomaterial nach Anspruch 3, wobei die Zelle eine für prokaryotische Expressionsvektoren geeignete *Escherichia-coli-Zelle* oder eine für eukaryotische Expressionsvektoren geeignete *Saccharomyces-cerevisiae-Zelle* ist.

6. Ein UGT-Protein, wobei das UGT-Protein die in SEQ ID NO. 8 gezeigte Aminosäuresequenz aufweist.

7. Verwendung des Enzymgens, das eine Nukleotidsequenz gemäß SEQ ID NO. 4 oder eines Proteins, das eine Aminosäuresequenz gemäß SEQ ID NO. 8 kodiert, aufweist, oder des Enzymgens, das eine Nukleotidsequenz aufweist, die mindestens 92 % Identität mit der Nukleotidsequenz gemäß SEQ ID NO. 4 aufweist, oder des Enzymgens, das ein Protein kodiert, dessen Aminosäuresequenz mindestens 92 % Identität mit der in SEQ ID NO. 8 gezeigten Aminosäuresequenz aufweist, oder des Biomaterials gemäß einem der Ansprüche 3-5, oder des Proteins, das von der in SEQ ID NO. 4 gezeigten Nukleotidsequenz kodiert wird, bei der mikrobiellen industriellen Synthese von Crocinen.

8. Die Verwendung gemäß Anspruch 7, wobei die Verwendung die Anwendung eines UGT-Proteins mit der in SEQ ID NO. 8 gezeigten Aminosäuresequenz bei der Synthese von Crocinen I-V aus Crocetin durch Glykosylierung ist.

9. Ein Verfahren zur Synthese von Crocinen, wobei das Verfahren den folgenden Schritt umfasst:
Verwendung eines UGT-Proteins mit der in SEQ ID NO. 8 gezeigten Aminosäuresequenz zur Glykosylierung von Crocetin unter Bildung von Crocin.

## Revendications

1. Gène enzymatique pour la biosynthèse des crocines, dans lequel la séquence d'acides aminés d'une protéine codée par le gène enzymatique est telle que représentée dans la SEQ ID NO. 8, ou est une séquence d' s présentant au moins 92 % d'identité avec la séquence d'acides aminés représentée dans la SEQ ID NO. 8.

2. Gène d'enzyme selon la revendication 1, dans lequel la séquence nucléotidique du gène d'enzyme est telle que représentée dans la SEQ ID NO. 4, ou est une séquence présentant au moins 92 % d'identité avec la séquence nucléotidique représentée dans la SEQ ID NO. 4.

3. Biomatériau constituant un vecteur d'expression, une cellule, une lignée cellulaire, une cassette d'expression ou un micro-organisme génétiquement modifié, choisi parmi *Escherichia coli* et *Saccharomyces cerevisiae,* contenant un gène d'enzyme dont la séquence nucléotidique est telle que représentée dans la SEQ ID NO. 4 ou dont la séquence d'acides aminés d'une protéine codée est telle que représentée dans la SEQ ID NO. 8, ou un gène d'enzyme dont la séquence nucléotidique présente une identité d'au moins 92 % avec la séquence nucléotidique représentée dans la SEQ ID NO. 4, ou un gène d'enzyme codant pour une protéine présentant une identité d'au moins 92 % avec la séquence d'acides aminés représentée dans la SEQ ID NO. 8.

4. Biomatériau selon la revendication 3, dans lequel le vecteur d'expression est l'un ou plusieurs parmi les vecteurs d'expression procaryotes de la série pET32a, pET28a et pCold I, et les vecteurs d'expression eucaryotes de la série pESC.

5. Biomatériau selon la revendication 3, dans lequel la cellule est une cellule *d'Escherichia coli* adaptée aux vecteurs d'expression procaryotes, ou une cellule *d' u de Saccharomyces cerevisiae* adaptée aux vecteurs d'expression eucaryotes.

6. Protéine UGT, dans laquelle 1' e de la protéine UGT présente la séquence d'acides aminés indiquée dans la SEQ ID NO. 8.

7. Utilisation du gène enzymatique présentant une séquence nucléotidique telle que représentée dans SEQ ID NO. 4 ou une séquence d'acides aminés d'une protéine codée telle que représentée dans SEQ ID NO. 8, ou du gène enzymatique présentant une séquence nucléotidique ayant au moins 92 % d'identité avec la séquence nucléotidique telle que représentée dans SEQ ID NO. 4, ou le gène d'enzyme codant pour une protéine présentant au moins 92 % d'identité avec la séquence d'acides aminés indiquée dans la SEQ ID NO. 8, ou le biomatériau selon l'une quelconque des revendications 3 à 5, ou la protéine codée par la séquence nucléotidique indiquée dans la SEQ ID NO. 4 , dans la synthèse industrielle microbienne de crocines.

8. Utilisation selon la revendication 7, dans laquelle l'utilisation consiste en l'application d'une protéine UGT ayant la séquence d'acides aminés indiquée dans SEQ ID NO. 8 dans la synthèse des crocines I à V à partir de la crocétine par glycosylation.

9. Procédé de synthèse de crocines, **caractérisé en ce qu'**il comprend l'étape suivante :
l'utilisation d'une protéine UGT présentant la séquence d'acides aminés indiquée dans SEQ ID NO. 8 pour glycosyler la crocétine afin de produire de la crocine.
